# EUROPÄISCHE PATENTSCHRIFT

(11) **EP 3 119 281 B1**
(45) Veröffentlichungstag und Bekanntmachung des Hinweises auf die Patenterteilung: **13.03.2024**
(21) Anmeldenummer: 15712835.6
(22) Anmeldetag: 19.03.2015
(51) Int. Cl.: A61B 5/1172, A61B 5/00

(54) **VORRICHTUNG UND VERFAHREN ZUR OPTISCHEN ERFASSUNG EINER OBERFLÄCHENSTRUKTUR UND VERFAHREN ZUR HERSTELLUNG EINER SOLCHEN VORRICHTUNG**
DEVICE AND METHOD FOR OPTICALLY DETECTING A SURFACE STRUCTURE, AND METHOD FOR PRODUCING SUCH A DEVICE
DISPOSITIF ET PROCÉDÉ DE SAISIE OPTIQUE D'UNE STRUTURE DE SURFACE ET PROCÉDÉ DE FABRICATION D'UN TEL DISPOSITIF

(30) Priorität: 21.03.2014 DE 102014205362
(43) Veröffentlichungstag der Anmeldung: 25.01.2017
(73) Patentinhaber: Bundesdruckerei GmbH, 10969 Berlin (DE)
(72) Erfinder: RABELER, Uwe, 30453 Hannover (DE); WOLF, Andreas, 07743 Jena (DE)
(74) Vertreter: Ramrath, Lukas
(86) Internationale Anmeldenummer: PCT/EP2015/055750
(87) Internationale Veröffentlichungsnummer: WO 2015/140238

(56) Entgegenhaltungen:
- EP-A1- 2 555 137
- CN-A- 103 019 474
- CN-U- 202 632 318
- DE-A1-102009 055 737
- JP-A- H1 153 524
- JP-A- 2009 105 601
- JP-A- 2009 165 731
- JP-A- 2009 172 263
- US-A1- 2009 232 362
- US-A1- 2010 220 299
- US-A1- 2010 322 550
- US-A1- 2011 122 405

## Beschreibung

Die Erfindung betrifft eine Vorrichtung zur optischen Erfassung einer Oberflächenstruktur eines Prüfkörpers, insbesondere eines Fingers. Weiter betrifft die Erfindung ein Verfahren zur optischen Erfassung einer Oberflächenstruktur eines Prüfkörpers sowie ein Verfahren zur Herstellung der vorhergehend angeführten Vorrichtung.

Fingerabdruckscanner mit einer hohen Erfassungsqualität, insbesondere solche, die anhand der Kriterien aus EBTS/F des FBI zertifizierbar sind, beruhen bis heute fast ausschließlich auf dem Prinzip der gestörten Totalreflexion. Dieses Funktionsprinzip aber bedingt die Verwendung eines Prismas, um eine optische Erfassung des Fingers zu ermöglichen. Geometrische Abmaße, insbesondere Dicken, der verwendeten Prismen können zwar reduziert werden, jedoch ist eine solche Reduktion nicht beliebig möglich.

Auch bekannt sind Ansätze, einen Fingerabdruck durch direktes Abfotografieren des Fingers zu erfassen. Ein Hauptproblem besteht hierbei in dem erheblichen algorithmischen Aufwand, insbesondere darin, trotz des geringen Kontrastes zwischen Bergen und Täler des Papillarlinienmusters auf der Fingerspitze eine zuverlässige optische Erfassung zu ermöglichen.

Die JP 2009 165731 A offenbart eine Vorrichtung zur Erfassung von biologischen Informationen. Insbesondere wird eine Venen-Authentifizierungs-Vorrichtung mit einer Bildeinheit, einer Lichtquelle und einem Mikrolinsenarray-Substrat beschrieben.

Die JP 2009 172263 A offenbart ebenfalls eine Vorrichtung zur Erfassung von biologischen Informationen. Insbesondere wird beschrieben, dass Licht einer Lichtquelle über eine Lichtleitplatte in einen Finger gestrahlt wird. Auch diese Vorrichtung dient aber zur Authentifizierung von Venen.

Die US 2010/322550 A1 offenbart ein Gerät zur Erfassung biometrischer Informationen und ein Gerät zur biometrischen Authentifizierung.

Die DE 10 2009 055 737 A1 offenbart eine optische Vorrichtung zur Erzeugung einer durch ein Objekt störfähigen internen Totalreflexion und deren Verwendung als Bild- oder Flächensensor, beispielsweise zur Detektion von Hand- oder Fingerabdrücken.

Es stellt sich das technische Problem, eine Vorrichtung und ein Verfahren zur optischen Erfassung einer Oberflächenstruktur eines Prüfkörpers, insbesondere eines Fingers, zu schaffen, welche eine zuverlässige optische Erfassung ermöglichen und gleichzeitig einen benötigten Bauraum der Vorrichtung reduzieren. Ein weiteres technisches Problem besteht darin, ein Verfahren zur Herstellung einer derartigen Vorrichtung zu schaffen.

Die Lösung des technischen Problems ergibt sich aus den Gegenständen mit den Merkmalen der Ansprüche 1, 8 und 11. Weitere vorteilhafte Ausgestaltungen der Erfindung ergeben sich aus den Unteransprüchen.

Es ist eine Grundidee der Erfindung, Licht in ein flach ausgebildetes Lichtleitelement, insbesondere ein folienartiges Lichtleitelement, einzukoppeln und das eingekoppelte Licht an einer ersten Oberfläche zur Beleuchtung des Prüfkörpers auszukoppeln, wobei das ausgekoppelte Licht nur in das Lichtleitelement zurückreflektiert wird, falls ein Teil des Prüfkörpers an dieser Stelle direkt auf der ersten Oberfläche aufliegt. Dieses reflektierte Licht kann dann optisch erfasst werden, um das gewünschte Abbild der Oberflächenstruktur des Prüfkörpers zu erzeugen.

Vorgeschlagen wird eine Vorrichtung zur optischen Erfassung einer Oberflächenstruktur eines Prüfkörpers. Die Vorrichtung dient insbesondere zur optischen Erfassung eines Fingers oder Fingerabdrucks und kann in diesem Fall auch als Fingerabdrucksensor oder -scanner bezeichnet werden.

Die Vorrichtung umfasst mindestens eine Beleuchtungseinrichtung. Mittels der Beleuchtungseinrichtung kann Licht mit einem vorbestimmten Spektrum erzeugt werden. Die Beleuchtungseinrichtung kann hierbei ein beliebiges Leuchtmittel, insbesondere aber zumindest eine Beleuchtungsquelle, z.B. eine LED oder eine ähnliche Beleuchtungsquelle, aufweisen, um Licht zu erzeugen.

Weiter umfasst die Vorrichtung mindestens ein Lichtleitelement. Das Lichtleitelement bezeichnet hierbei ein Element, in dem Licht der Beleuchtungseinrichtung geführt oder geleitet werden kann. Insbesondere kann das Lichtleitelement aus einem Material oder einer Materialmischung bestehen, das/die einen vorbestimmten Brechungsindex und/oder eine vorbestimmte Ausbreitungsgeschwindigkeit für Licht aufweist. Das Lichtleitelement kann insbesondere flach ausgebildet sein, d.h. eine geringe geometrische Höhe bzw. Dicke aufweisen. Insbesondere kann das Lichtleitelement als Folie ausgebildet sein.

Weiter ist mittels der Beleuchtungseinrichtung Licht in das Lichtleitelement einkoppelbar. Hierfür können das Lichtleitelement, insbesondere ein Einkoppelbereich des Lichtleitelements, und die mindestens eine Beleuchtungseinrichtung optisch derart verbunden sein, dass von der Beleuchtungseinrichtung erzeugtes Licht in das Lichtleitelement eingekoppelt werden kann. Insbesondere kann die Beleuchtungseinrichtung unmittelbar an einer äußeren Fläche, insbesondere einer Seitenfläche, weiter insbesondere an einem seitlichen Rand, des Lichtleitelements angeordnet sein.

Weiter weist das Lichtleitelement eine erste Oberfläche auf. Die erste Oberfläche kann hierbei vorbestimmte geometrische Abmaße, insbesondere eine vorbestimmte Länge und eine vorbestimmte Breite, aufweisen. Auch kann die erste Oberfläche eine vorbestimmte Struktur, insbesondere eine vorbestimmte Oberflächenstruktur, aufweisen. Weiter ist Licht im Bereich der ersten Oberfläche aus dem Lichtleitelement auskoppelbar. Dies kann bedeuten, dass Licht aus dem Lichtleitelement durch die erste Oberfläche austritt.

Erfindungsgemäß ist das Lichtleitelement im Bereich der ersten Oberfläche oder in zumindest einem Teilbereich der ersten Oberfläche für einen Luft-Lichtleitelement-Übergang für das ausgekoppelte und zurückreflektierte Licht, insbesondere für einen Teil mit einem vorbestimmten Spektrum, vollständig reflektierend. Der Luft-Lichtleitelement-Übergang bezeichnet hierbei einen Übergang von Luft in das Lichtleitelement. Somit wird also Licht, welches von außen auf die erste Oberfläche trifft, vollständig reflektiert, d.h. total reflektiert, falls ein Luft-Lichtleitelement-Übergang an der Stelle vorliegt, an der das Licht auf das Lichtleitelement auftrifft. Weiter ist das Lichtleitelement im Bereich der ersten Oberfläche für das ausgekoppelte und zurückreflektierte Licht bei einem Medium-Lichtleitelement-Übergang unvollständig reflektierend, wobei das Medium ein von Luft verschiedenes Medium ist. Dies kann bedeuten, dass Licht, welches von außen auf die erste Oberfläche des Lichtleitelements trifft, unvollständig, insbesondere nicht, reflektiert wird und somit zumindest teilweise oder vollständig in das Lichtleitelement eintritt, falls ein Medium-Luft-Übergang an der Stelle vorliegt, an der das Licht auf das Lichtleitelement auftrifft.

Hierbei kann das Medium ein Medium mit einem vorbestimmten Brechungsindex und einer vorbestimmten Ausbreitungsgeschwindigkeit für Licht sein, wobei der Brechungsindex und die Ausbreitungsgeschwindigkeit des Mediums von dem Brechungsindex bzw. von der Ausbreitungsgeschwindigkeit von Luft verschieden ist/sind. Das Medium kann auch ein Medium sein, dessen Brechungsindex in einem vorbestimmten Bereich von Brechungsindizes und/oder dessen Ausbreitungsgeschwindigkeit in einem vorbestimmten Bereich von Ausbreitungsgeschwindigkeiten liegt.

Insbesondere ist die erste Oberfläche eine ebene und ungekrümmte Oberfläche. Die erste Oberfläche bildet weiter eine Auflagefläche für den zu erfassenden Prüfkörper aus. In diesem Fall ergibt sich, dass im Bereich der ersten Oberfläche aus dem Lichtleitelement ausgekoppeltes Licht, welches außerhalb des Lichtleitelements auf den Prüfkörper trifft und von diesem zurück zur ersten Oberfläche reflektiert oder gestreut wird, nur dann durch die erste Oberfläche in das Lichtleitelement eintritt, falls ein Medium-Lichtleitelement-Übergang vorliegt, insbesondere in dem Bereich oder an der Stelle, in dem/an der das Licht auf die erste Oberfläche auftrifft. Das vorhergehend erläuterte Medium kann somit insbesondere einem Medium eines zu prüfenden Prüfkörpers entsprechen.

Falls ein Luft-Lichtleitelement-Übergang vorliegt, so kann das vom Prüfkörper zurückreflektierte oder zurückgestreute Licht nicht in das Lichtleitelement eintreten. Ein Luft-Lichtleitelement-Übergang liegt insbesondere dann vor, wenn ein Teilbereich einer Oberfläche des Prüfkörpers nicht unmittelbar an der ersten Oberfläche anliegt, beispielsweise weil der Prüfkörper in diesem Bereich eine (relativ zur Auflagefläche oder ersten Oberfläche gesehene) Vertiefung aufweist. Ist der Prüfkörper ein Finger, so tritt Licht, welches aus dem Lichtleitelement ausgekoppelt und von dem Finger zurückgestreut oder -reflektiert wird, nur in den Bereichen durch die erste Oberfläche in das Lichtleitelement ein, in denen der Finger unmittelbar auf der ersten Oberfläche aufliegt, insbesondere also in Bereichen von Papillarleisten. Im Bereich von Vertiefungen des Fingers, insbesondere von Rillen z.B. zwischen Papillarleisten, tritt kein zurückgestreutes oder -reflektiertes Licht in das Lichtleitelement ein.

Weiter kann die Vorrichtung mindestens eine Bilderfassungseinrichtung umfassen. Diese kann derart angeordnet und/oder ausgebildet sein, dass der an dem Medium-Lichtleitelement-Übergang nicht reflektierte Anteil des Lichts optisch erfassbar ist. Insbesondere kann also die Bilderfassungseinrichtung den nicht an der ersten Oberfläche reflektierten Anteil des von dem Prüfkörper zurückgestreuten oder-reflektierten Lichts erfassen.

Da das Lichtleitelement geometrisch flach ausgeführt werden kann, ergibt sich in vorteilhafter Weise eine qualitativ hochwertige und zuverlässige optische Erfassung einer Oberflächenstruktur des Prüfkörpers, insbesondere eines Fingerabdrucks, wobei ein Bauraumbedarf reduziert wird. Durch erfindungsgemäß ermöglichte kompakte Ausbildung einer optischen Erfassungsvorrichtung kann diese einfach in bestehende Systeme, beispielsweise Terminals am Flughafen, integriert werden.

In einer weiteren Ausführungsform ist das Lichtleitelement derart ausgebildet, dass die erste Oberfläche mindestens einen, vorzugsweise jedoch mehrere, Auskoppelbereich(e) aufweist, wobei nur in dem mindestens einen Auskoppelbereich Licht aus dem Lichtleitelement auskoppelbar ist. Ein Auskoppelbereich ist hierbei ein Teilbereich der ersten Oberfläche und kann somit insbesondere geometrische Abmaße aufweisen, die kleiner als die korrespondierenden geometrischen Abmaße der ersten Oberfläche sind. Somit kann also nur in diskreten Bereichen Licht aus dem Lichtleitelement ausgekoppelt werden. Dies wiederum führt dazu, dass die Oberfläche eines Prüfkörpers nur in bestimmten Bereichen beleuchtet wird, wobei somit nur Licht, welches in diesen Bereichen austritt, von der Oberfläche des Prüfkörpers zurückreflektiert oder -gestreut werden kann. Hierdurch ist es z.B. möglich, nur bestimmte Teilbereiche der Oberfläche des Prüfkörpers zu beleuchten. Hierdurch wiederum lässt sich ein Kontrast bei der optischen Erfassung verbessern, da eine Überlagerung der von verschiedenen Bereichen der Oberfläche des Prüfkörpers zurückreflektierten oder -gestreuten Strahlung verringert wird.

In einer weiteren Ausführungsform weist das Lichtleitelement eine weitere Oberfläche auf, wobei die weitere Oberfläche eine der ersten Oberfläche gegenüberliegende Oberfläche des Lichtleitelements ist, wobei der mindestens eine Auskoppelbereich durch eine Ausbildung, insbesondere eine Oberflächenstruktur, der weiteren Oberfläche festgelegt ist.

Durch die Festlegung der Auskoppelbereiche an der ersten Oberfläche durch die Ausbildung, insbesondere die Ausbildung einer Oberflächenstruktur, der zweiten Oberfläche kann vorteilhafter Weise vermieden werden, die vorzugsweise ebene Oberflächenstruktur der ersten Oberflächenstruktur zu verändern. Auch ist nicht erforderlich, im Bereich der ersten Oberfläche zusätzliche optische Elemente, z.B. Filterelemente oder Abdeckelemente, vorzusehen.

In einer weiteren Ausführungsform ist eine Oberflächenstruktur der weiteren Oberfläche derart ausgebildet, dass nur in mindestens einem, vorzugsweise aber mehreren, vorbestimmten Bereich(en) der weiteren Oberfläche das mittels der Beleuchtungseinrichtung eingekoppelte Licht derart in Richtung der ersten Oberfläche lenkbar ist, dass es durch die erste Oberfläche aus dem Lichtleitelement austritt. Insbesondere ist der mindestens eine vorbestimmte Bereich der weiteren Oberfläche derart ausgebildet, dass Licht nur in dem mindestens einen Auskoppelbereich durch die erste Oberfläche austritt.

Das Lichtleitelement kann hierbei derart ausgebildet sein, dass Licht nur dann durch die erste Oberfläche aus dem Lichtleitelement austreten kann, falls das Licht mit einem vorbestimmten Winkel oder mit einem Winkel aus einem vorbestimmten Winkelbereich, beispielsweise zwischen 70° und 110°, relativ zur ersten Oberfläche auf die erste Oberfläche auftrifft.

Hierdurch ergibt sich in vorteilhafter Weise, dass die Auskoppelbereiche auf der ersten Oberfläche durch eine geometrische Ausbildung der Oberflächenstruktur der weiteren Oberfläche festgelegt werden können. Dies wiederum bedingt, wie nachfolgend näher erläutert, eine vereinfachte Herstellung des Lichtleitelements.

Weiter weist die weitere Oberfläche mindestens einen erhabenen Bereich auf. Insbesondere weist die weitere Oberfläche mehrere erhabene Bereiche auf. Dies kann bedeuten, dass die weitere Oberfläche mindestens zwei, vorzugsweise jedoch mehr als zwei, erhabene Bereiche aufweist, wobei zwischen den mindestens zwei erhabenen Bereichen ein Zwischenbereich angeordnet ist. Der Zwischenbereich bildet somit eine Vertiefung zwischen erhabenen Bereichen. Ein erhabener Bereich kann einen von einer Grundfläche der weiteren Oberfläche hervorstehenden Bereich bezeichnen.

Die erhabenen Bereiche können hierbei eine beliebige geometrische Form aufweisen. Insbesondere können die erhabenen Bereiche sphärisch, kubisch oder pyramidisch ausgebildet sein. Vorzugsweise weisen die erhabenen Bereiche eine (teil-)zylinderförmige Form auf. Eine freiliegende Stirnfläche des Zylinders kann hierbei eben oder (in Bezug auf die weitere Oberfläche) konkav oder konvex gewölbt sein oder als Freiform ausgebildet sein. Auch weitere Formen der Oberfläche der freiliegenden Stirnseite sind selbstverständlich vorstellbar.

Hierdurch ergibt sich in vorteilhafter Weise, dass Licht, welches durch die mindestens eine Beleuchtungseinrichtung in das Lichtleitelement einkoppelbar ist, nur in den erhabenen Bereichen derart umgelenkt wird, dass es durch die erste Oberfläche aus dem Lichtleitelement austreten kann. Die gewünschte optische Strahllenkung wird hierdurch in vorteilhafter Weise durch die Ausbildung der Oberflächenstruktur der weiteren Oberfläche erreicht.

Erfindungsgemäß weist die weitere Oberfläche mindestens zwei erhabene Bereiche auf, wobei zwischen den mindestens zwei erhabenen Bereichen ein Zwischenbereich angeordnet ist, wobei in dem Zwischenbereich ein lichtundurchlässiges Element angeordnet ist. Insbesondere kann das lichtundurchlässige Element den Zwischenbereich vollständig ausfüllen. Hierdurch wird in vorteilhafter Weise eine Verschmutzung des Lichtleitelements im Bereich der weiteren Oberfläche verhindert.

Das lichtundurchlässige Element kann insbesondere undurchlässig für Licht mit einem vorbestimmten Spektrum, beispielsweise für zumindest einen Teil des von der Beleuchtungseinrichtung erzeugten Lichts, sein.

Weiter wird durch die Anordnung eines lichtundurchlässigen Elements im Zwischenbereich gewährleistet, dass von dem Prüfkörper zurückreflektiertes oder -gestreutes Licht, welches durch die erste Oberfläche in das Lichtleitelement eintritt, im Bereich der weiteren Oberfläche nur durch die erhabenen Bereiche wieder aus dem Lichtleitelement austreten kann. Dies vereinfacht die optische Erfassung des vorhergehend erläuterten, nicht von der ersten Oberfläche reflektierten Lichts, insbesondere durch eine Bilderfassungseinrichtung. Diese kann hierfür beispielsweise auf der Seite der weiteren Oberfläche angeordnet sein, wobei die weitere Oberfläche im Erfassungsbereich der Bilderfassungseinrichtung liegt.

Somit wird durch die Anordnung der Zwischenbereiche eine optische Auflösung der Vorrichtung beeinflusst.

In einer weiteren Ausführungsform weist das Lichtleitelement im Bereich der weiteren Oberfläche mindestens ein optisches Element auf. Das optische Element kann hierbei ein Element zur Strahlführung, insbesondere zur Strahllenkung, sein. Beispielsweise kann das optische Element eine Linsenfunktion aufweisen. Weiter insbesondere kann das optische Element durch eine Ausbildung bzw. eine geometrische Form des erhabenen Bereichs ausgebildet oder bereitgestellt werden.

Hierdurch kann in vorteilhafter Weise die erforderliche Leistung der Lichtquellen reduziert werden.

Weiter kann die Beleuchtung des Prüfkörpers oder eines vorbestimmten Teils des Prüfkörpers mit mindestens einem vorbestimmten Beleuchtungsparameter erfolgen. Ein Beleuchtungsparameter kann beispielsweise eine vorbestimmten Beleuchtungsrichtung, eine vorbestimmte Polarisation, eine vorbestimmte Lichtfarbe oder ein weiterer vorbestimmter Beleuchtungsparameter sein. Hierbei ist es möglich, dass der mindestens eine vorbestimmte Beleuchtungsparameter zumindest teilweise durch das mindestens eine optische Element eingestellt wird.

In einer weiteren Ausführungsform sind die Auskoppelbereiche derart angeordnet, dass eine optische Erfassung mit einer vorbestimmten Auflösung möglich ist. Alternativ oder kumulativ können auch die erhabenen Bereiche der weiteren Oberfläche derart angeordnet und/oder ausgebildet sein, dass die optische Erfassung mit einer vorbestimmten Auflösung möglich ist.

Die optische Auflösung beträgt vorzugsweise mindestens 500 dpi. Insbesondere sind auch Auflösungen von 1.000 dpi oder andere, insbesondere noch höhere, Auflösungen vorstellbar, wobei diese abhängig von der zur Verfügung stehenden Bilderfassungstechnik und Fertigungsmöglichkeiten für das vorgeschlagene Lichtleiterelement sein können. Dies kann bedeuten, dass die Auskoppelbereiche und/oder die erhabenen Bereiche derart angeordnet sind, dass eine Bilderfassungseinrichtung eine Abbildung mit der gewünschten Auflösung von der Oberflächenstruktur des Prüfkörpers erzeugen kann.

Insbesondere können die erhabenen Bereiche und die Zwischenbereiche derart ausgebildet und/oder angeordnet sein, dass eine gewünschte Anzahl von erhabenen Bereichen und Zwischenbereichen entlang eines Wegs mit vorbestimmter Länge vorhanden ist. Beispielsweise können 500 erhabene Bereiche und die dazu gehörigen Zwischenbereiche in einem Inch angeordnet sein.

Bevorzugt können die Auskoppelbereiche bzw. die erhabenen Bereiche derart angeordnet und/oder ausgebildet sein, dass eine optische Erfassung mit 20 Linienpaaren pro Millimeter bei einem vorbestimmten Kontrast möglich ist. Ein Kontrast bezeichnet hier ein minimales Verhältnis zwischen einem Intensitätsminimum, welches beispielsweise einer Vertiefung im Bereich des Prüfkörpers entspricht bzw. diese im Abbild repräsentiert, und einen Intensitätsmaximum, welches beispielsweise einem aufliegenden Bereich des Prüfkörpers entspricht bzw. diese im Abbild repräsentiert. Der vorbestimmte Kontrast kann insbesondere 20 % betragen.

In einer weiteren Ausführungsform weist das Lichtleitelement eine maximale Höhe auf. Eine Höhe kann hierbei einen Abstand zwischen der weiteren Oberfläche, insbesondere zwischen einer Grundfläche oder zwischen einem freien Ende eines erhabenen Bereichs, und der ersten Oberfläche des Lichtleitelements bezeichnen, welche beispielsweise entlang einer Richtung senkrecht zur ersten Oberfläche gemessen wird. Eine maximale Höhe kann beispielsweise 4 mm betragen. Insbesondere kann das Lichtleitelement folienartig ausgebildet sein.

Hierdurch ergibt sich in vorteilhafter Weise eine besonders einfache Ausbildung des Lichtleitelements mit geringen geometrischen Abmaßen.

Weiter vorgeschlagen wird ein Verfahren zur Erfassung einer Oberflächenstruktur eines Prüfkörpers, insbesondere eines Fingers.

Das vorgeschlagene Verfahren kann hierbei insbesondere mittels einer Vorrichtung gemäß einer der vorhergehend beschriebenen Ausführungsformen durchführbar sein.

Das Verfahren umfasst folgende Verfahrensschritte: Der Prüfkörper wird auf eine erste Oberfläche eines Lichtleitelements aufgelegt, wobei Licht in das Lichtleitelement eingekoppelt wird. Weiter wird Licht im Bereich der ersten Oberfläche aus dem Lichtleitelement hin zum Prüfkörper ausgekoppelt. Weiter ist das Lichtleitelement im Bereich der ersten Oberfläche bei einem Luft-Lichtleitelement-Übergang für zumindest einen Teil des durch die Beleuchtungseinrichtung in das Lichtleitelement eingekoppelten Lichts vollständig reflektierend. Dies wiederum bedeutet, dass das Lichtleitelement im Bereich der ersten Oberfläche bei einem Luft-Lichtleitelement-Übergang für zumindest einen Teil des durch die Beleuchtungseinrichtung eingekoppelten Lichts und im Bereich der ersten Oberfläche ausgekoppelten und von dem Prüfkörper zurück zur ersten Oberfläche reflektierten oder gestreuten Lichts vollständig reflektierend ist. Bei einem Medium-Lichtleitelement-Übergang ist das Lichtleitelement im Bereich der ersten Oberfläche unvollständig reflektierend, wobei das Medium ein von Luft verschiedenes Medium ist. Weiter wird ein nicht an der ersten Oberfläche reflektierter Anteil des Lichtes, insbesondere der nichtreflektierte und hierdurch wieder durch die erste Oberfläche in das Lichtleitelement eintretende Anteil, erfasst.

Hierdurch ergibt sich in vorteilhafter Weise eine einfache, jedoch optisch qualitativ hochwertige Erfassung der Oberflächenstruktur des Prüfkörpers, insbesondere eines Fingerabdrucks.

In einer weiteren Ausführungsform wird Licht nur in mindestens einem Auskoppelbereich, vorzugsweise in mehreren Auskoppelbereichen, der ersten Oberfläche aus dem Lichtleitelement ausgekoppelt. Hierdurch kann ein Kontrast bei der optischen Erfassung verbessert werden.

In einer weiteren Ausführungsform wird in Abhängigkeit des erfassten Anteils, also des nicht an der ersten Oberfläche reflektierten Anteils, eine Lebenderkennung durchgeführt. Hierzu kann beispielsweise Licht zur Durchführung eines Puls-Oxymetrie-Verfahrens in das Lichtleitelement eingekoppelt werden. Hierzu kann Licht mit dem Fachmann bekannten Wellenlängen in das Lichtleitelement eingekoppelt werden. Beispielsweise kann Licht mit einer Wellenlänge von 660nm und/oder Licht
mit einem Spektrum im nahen Infrarotbereich, beispielsweise Licht mit einer Wellenlänge im Bereich von 940 nm bis 950nm in das Lichtleitelement eingekoppelt werden. Dieses Licht wird dann im Bereich der ersten Oberfläche ausgekoppelt und kann in einen durchbluteten Prüfkörper, insbesondere einen Finger, eindringen, wobei es von Blutgefäßen, insbesondere Venen, reflektiert werden kann. Zumindest ein Teil des reflektierten Lichts kann optisch erfasst werden, wobei in Abhängigkeit dieses erfassten Lichtes dann das bekannte Puls-Oxymetrie-Verfahren durchgeführt werden kann. Hierbei kann z.B. durch Überlagerung oder Differenzbildung mehrerer, zeitlich aufeinander folgenden optischen Aufnahmen, auf eine Blutzirkulation geschlossen werden.

Auch kann in Abhängigkeit des erzeugten und eingekoppelten Lichts eine Sauerstoffsättigung von im Blut enthaltenen Hämoglobins bestimmt werden. Hierzu kann beispielsweise blaues Licht, insbesondere Licht mit einer oder mehreren Wellenlängen aus einem Bereich von 400 bis 500 nm, eingekoppelt werden.

Hierdurch ergibt sich in vorteilhafter Weise eine verbesserte Prüfung von Fingerabdrücken.

Weiter vorgeschlagen wird ein Verfahren zur Herstellung einer Vorrichtung zur Erfassung einer Oberflächenstruktur eines Prüfkörpers, insbesondere eines Fingers. Das Verfahren kann hierbei zur Herstellung einer Vorrichtung gemäß einer der vorhergehend beschriebenen Ausführungsformen dienen. Hierbei werden mindestens eine Beleuchtungseinrichtung und mindestens ein Lichtleitelement bereitgestellt. Die Beleuchtungseinrichtung und das mindestens eine Lichtleitelement werden derart optisch verbunden, dass Licht der Beleuchtungseinrichtung in das Lichtleitelement einkoppelbar ist. Das Lichtleitelement wird derart bereitgestellt, dass das Lichtleitelement eine erste Oberfläche aufweist, wobei Licht im Bereich der ersten Oberfläche aus dem Lichtleitelement auskoppelbar ist. Weiter ist das Lichtleitelement im Bereich der ersten Oberfläche für einen Luft-Lichtleitelement-Übergang für zumindest einen Teil des von der Beleuchtungseinrichtung erzeugten Lichts vollständig reflektierend und bei einem Medium-Lichtleitelement-Übergang unvollständig reflektierend, wobei das Medium ein von Luft verschiedenes Medium ist. Hierdurch ergibt sich in vorteilhafter Weise ein Verfahren zur Herstellung einer Vorrichtung zur optischen Erfassung eines Prüfkörpers mit minimalen Bauraumbedarf.

In einer weiteren Ausführungsform wird eine weitere Oberfläche des Lichtleitelements bereitgestellt, wobei mindestens ein Auskoppelbereich der ersten Oberfläche durch eine Ausbildung der weiteren Oberfläche, insbesondere eine Ausbildung einer Oberflächenstruktur der weiteren Oberfläche, festgelegt ist. Die weitere Oberfläche kann hierbei insbesondere durch Prägen geformt und somit bereitgestellt werden. Hierdurch ergibt sich in vorteilhafter Weise eine einfache Herstellung der vorgeschlagenen Vorrichtung.

Die Erfindung wird anhand eines Ausführungsbeispiels näher erläutert. Die Figuren zeigen:
- Fig. 1: einen schematischen Querschnitt durch eine erfindungsgemäße Vorrichtung und einen korrespondierenden Intensitätsverlauf von reflektiertem Licht,
- Fig. 2: eine schematische Unteransicht des in Fig. 1 dargestellten Lichtleitelements und
- Fig. 3: eine schematische Seitenansicht einer erfindungsgemäßen Vorrichtung.

Nachfolgend bezeichnen gleiche Bezugszeichen Elemente mit gleichen oder ähnlichen technischen Merkmalen.

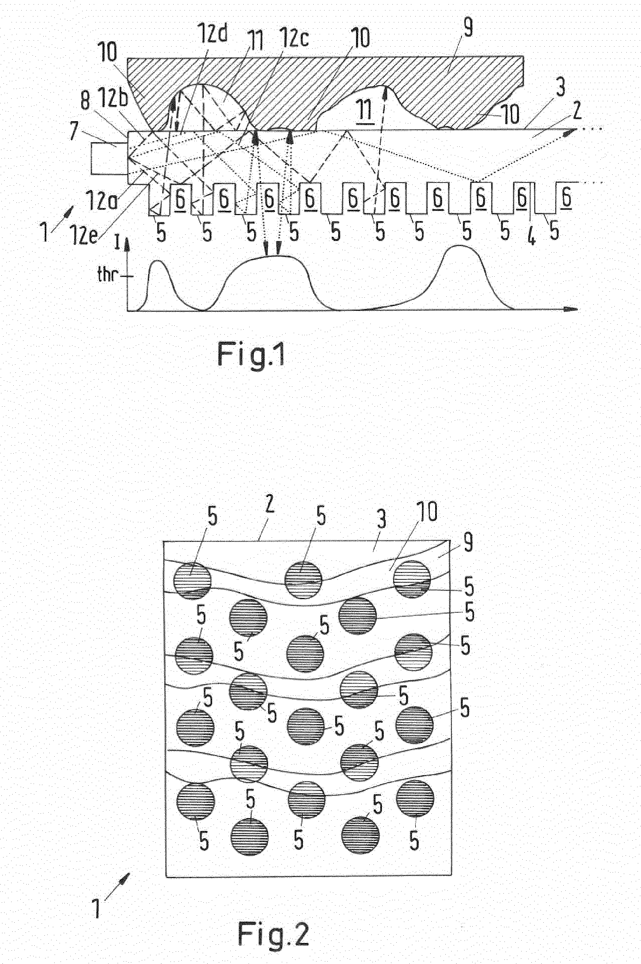

In Fig. 1 ist eine schematische Seitenansicht einer erfindungsgemäßen Vorrichtung 1 sowie ein korrespondierender Intensitätsverlauf I dargestellt. Die Vorrichtung 1 umfasst ein als Folie 2 ausgebildetes Lichtleitelement mit einer ersten Oberfläche 3 und einer weiteren Oberfläche 4. Die erste Oberfläche 3 ist als ebene und ungekrümmte Fläche ausgebildet. Die weitere Oberfläche 4 weist eine unebene Oberflächenstruktur auf. Insbesondere weist die weitere Oberfläche 4 zylinderförmige, erhabene Bereiche 5 auf. Zwischen den erhabenen Bereichen 5 sind Zwischenbereiche 6 angeordnet. Weiter umfasst die Vorrichtung 1 eine Beleuchtungseinrichtung 7, die an einem seitlichen Rand 8 der Folie 2 angeordnet ist. Von der Beleuchtungseinrichtung 7 erzeugtes Licht ist am seitlichen Rand 8 in die Folie 2 einkoppelbar. Die erste Oberfläche 3 bildet eine Auflagefläche für einen Finger 9 aus. Der Finger 9, der einen erfindungsgemäßen Prüfkörper darstellt, weist wiederum Papillarleisten 10 und zwischen Papillarleisten ausgebildete Vertiefungen 11 auf.

Die Folie 2 ist derart ausgebildet, dass die erste Oberfläche 3 für einen Luft-Folien-Übergang für das von der Beleuchtungseinrichtung 7 erzeugte Licht vollständig reflektierend und für einen Übergang von Haut zur Folie 2 nicht reflektierend ist.

Weiter dargestellt ist, dass Licht an der ersten Oberfläche 3 nur in bestimmten Auskoppelbereichen ausgekoppelt wird, wobei die Auskoppelbereiche durch Ausbildung der weiteren Oberfläche 4, insbesondere die Anordnung und Ausbildung der erhabenen Bereiche 5, festgelegt sind. Hierbei ist die Oberflächenstruktur der weiteren Oberfläche 4 durch die Ausbildung und Anordnung der erhabenen Bereiche 5 und der Zwischenbereiche 6 derart ausgebildet, dass nur im Bereich der erhabenen Bereiche 5 das mittels der Beleuchtungseinrichtung 7 eingekoppelte Licht derart in Richtung der ersten Oberfläche 3 gelenkt wird, dass es durch die erste Oberfläche 3 in Richtung des Fingers 9 austritt. Dies ist schematisch durch exemplarische Strahlengänge von Lichtstrahlen 12a, 12b, 12c, 12d, 12e dargestellt. Diese Lichtstrahlen werden durch die erhabenen Bereiche 5 insbesondere derart umgelenkt, dass sie mit einem Winkel aus einem vorbestimmten Winkelbereich, beispielsweise zwischen 70° und 110°, auf die erste Oberfläche 3 treffen und hierdurch durch die erste Oberfläche 3 aus der Folie 2 austreten können. Hierbei können nur Lichtstrahlen 12a, ..., 12e aus der Folie 2 austreten, wenn sie unter einem Winkel aus diesem Winkelbereich auf die erste Oberfläche 3 treffen.

Die Lichtstrahlen 12a, 12b, 12e die jeweils durch eine gestrichelte Linie dargestellt sind, treten hierbei in eine Vertiefung 11 ein und werden von den die Vertiefung 11 begrenzenden Oberflächen des Fingers 9 reflektiert bzw. gestreut und treffen wieder auf die erste Oberfläche 3. Aufgrund der optischen Ausbildung der Folie 2 können diese jedoch nicht durch die erste Oberfläche 3 in die Folie eintreten.

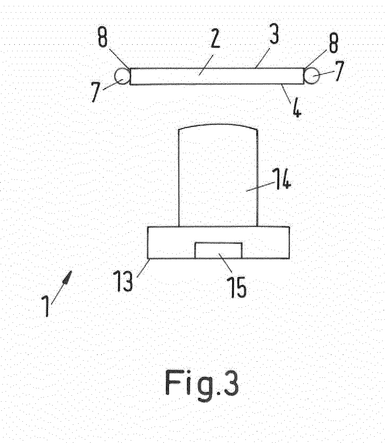

Im Gegensatz dazu treten Lichtstrahlen 12c, 12d, die jeweils durch einen gepunktete Linie dargestellt sind und die ebenfalls durch erhabene Bereiche 5 in Richtung der ersten Oberfläche 3 reflektiert wurden, im Bereich einer auf der ersten Oberfläche 3 aufliegenden Papillarleiste 10 aus der Folie 2 aus. Diese Lichtstrahlen 12c, 12d werden ebenfalls von der Oberfläche des Fingers 9 im Bereich der aufliegenden Papillarleiste 10 gestreut und reflektiert. Da in diesem Bereich ein Haut-Folien-Übergang vorliegt, können diese Lichtstrahlen wieder durch die erste Oberfläche 3 in die Folie 2 eintreten. Weiter ist dargestellt, dass die derart von dem Finger 9 reflektierten Lichtstrahlen 12c, 12d durch die Folie 2 hindurchgestrahlt werden und durch die weitere Oberfläche 4 aus der Folie 2 austreten. Hierbei kann, wie in Fig. 3 noch näher dargestellt, eine Bilderfassungseinrichtung 13 auf der Seite der weiteren Oberfläche 4 angeordnet sein und diese Lichtstrahlen 12c, 12d erfassen.

In Fig. 1 ist weiter ein zu der Oberflächenstruktur des Fingers 9 korrespondierender Intensitätsverlauf I dargestellt. Hierbei ist ersichtlich, dass die Intensität I in räumlichen Bereichen über einem vorbestimmten Schwellwert thr liegt bzw. ein Maximum aufweist, wobei die räumlichen Bereiche zu Bereichen korrespondieren, in denen auf der ersten Oberfläche 3 Papillarleisten 10 des Fingers 9 aufliegen. Somit ist eine optisch zuverlässige und mit ausreichendem Kontrast versehende optische Erfassung der Oberflächenstruktur des Fingers 9 möglich.

In Fig. 2 ist eine schematische Unteransicht auf die in Fig. 1 dargestellte Vorrichtung 1 dargestellt. Hierbei sind insbesondere die zylinderförmig erhabenen Bereiche 5, die von der in Fig. 1 dargestellten weiteren Oberfläche 4 der Folie 2 ausgebildet werden, ersichtlich. Weiter dargestellt sind Papillarleisten 10 des Fingers 9, die auf der Oberfläche 3 aufliegen.

Hierbei ist ersichtlich, dass die zylinderförmig erhabenen Bereiche 5 in einer regelmäßigen Struktur angeordnet sind. Ein Abstand zwischen den erhabenen Bereichen 5 kann beispielsweise ein Abstand zwischen zentralen Symmetrieachsen der zylinderförmigen Bereiche 5 sein, wobei der Abstand parallel zur ersten Oberfläche 3 gemessen wird. Der Abstand zwischen den erhabenen Bereichen 5 ist hierbei derart gewählt, dass eine gewünschte Reflexionsauflösung und somit, abhängig von Erfassungsparametern einer Bilderfassungseinrichtung, auch eine gewünschte optische Auflösung eines Abbildes erreichbar ist. Beispielsweise können die Abstände derart gewählt werden, dass eine optische Auflösung mit mindestens 500 dpi erreichbar ist.

In Fig. 3 ist eine schematische Seitenansicht einer erfindungsgemäßen Vorrichtung 1 dargestellt. Insbesondere dargestellt ist eine Folie 2, die entsprechend der in Fig. 1 dargestellten Ausführungsform ausgebildet sein kann. Die Folie 2 weist eine erste Oberfläche 3 und eine weitere Oberfläche 4 auf (siehe Fig. 1). Weiter umfasst die Vorrichtung 1 zwei Beleuchtungseinrichtungen 7, die jeweils an seitlichen Rändern 8 der Folie 2 angeordnet sind und beispielsweise unmittelbar an diesen seitlichen Rändern 8 anliegen. Weiter umfasst die Vorrichtung 1 eine Bilderfassungseinrichtung 13, die wiederum ein Objektiv 14 und ein Sensorelement 15, beispielsweise einen CCD-Sensor, umfasst. Die Bilderfassungseinrichtung 13 ist hierbei auf einer weiteren Oberfläche 4 der Folie 2 zugewandten Seite der Folie 2 angeordnet und erfasst somit das z.B. von einem Finger 9 (siehe Fig. 1) durch die Folie 2 reflektierte Licht.

Die Folie 2 kann insbesondere eine mikrooptische Struktur aufweisen. Die in den Fig. 1 bis 3 dargestellten Ausführungsformen ermöglichen die Erzeugung von räumlich definierten Pixelbereichen mit einem gewünschten, insbesondere optimalen, Kontrast zwischen Bereichen, die auf der Oberfläche 3 aufliegenden Papillarbereichen 10 und Vertiefungen 11 abbilden. Insbesondere kann nur Licht, welches von aufliegenden Papillarbereichen 10 reflektiert wird, optisch erfasst werden. Die Folie 2 kann in vorteilhafter Weise mit einer flachen Bauform ausgebildet werden. Es ist möglich, in den Zwischenbereichen 6 (siehe Fig. 1) eine lichtundurchlässige oder wie gewünscht verfärbte Zwischenschicht einzubringen.

Die dargestellte Vorrichtung 1 kann ebenfalls in eine Dokumentenauflage eines Dokumentenerfassungsgerätes, beispielsweise eines Passlesers, integriert werden, womit dieses ebenfalls in die Lage versetzt wird, Fingerabdrücke zu erfassen.

### Bezugszeichenliste

- 1: Vorrichtung
- 2: Folie
- 3: erste Oberfläche
- 4: weitere Oberfläche
- 5: erhabener Bereich
- 6: Zwischenbereich
- 7: Beleuchtungseinrichtung
- 8: seitlicher Rand
- 9: Finger
- 10: Papillarleiste
- 11: Vertiefung
- 12a, 12b, 12c, 12d, 12e: Lichtstrahl
- 13: Bilderfassungseinrichtung
- 14: Objektiv
- 15: Sensor
- thr: Schwellwert

## Patentansprüche

1. Vorrichtung zur optischen Erfassung einer Oberflächenstruktur eines Prüfkörpers, insbesondere eines Fingers (9), wobei die Vorrichtung (1) mindestens eine Beleuchtungseinrichtung (7) umfasst, wobei die Vorrichtung (1) mindestens ein Lichtleitelement umfasst, wobei mittels der Beleuchtungseinrichtung (7) Licht in das Lichtleitelement einkoppelbar ist, wobei das Lichtleitelement eine erste Oberfläche (3) aufweist, wobei Licht im Bereich der ersten Oberfläche (3) aus dem Lichtleitelement auskoppelbar ist, **dadurch gekennzeichnet, dass**
eine weitere Oberfläche (4) des Lichtleitelements (3) mindestens mindestens zwei erhabene Bereiche (5) aufweist, wobei zwischen den mindestens zwei erhabenen Bereichen ein Zwischenbereich angeordnet ist,
das Lichtleitelement im Bereich der ersten Oberfläche (3) für einen Luft-Lichtleitelement-Übergang für zumindest einen Teil des von der Beleuchtungseinrichtung (7) erzeugten Lichts vollständig reflektierend und bei einem Medium-Lichtleitelement-Übergang unvollständig reflektierend ist, wobei das Medium ein von Luft verschiedenes Medium ist, wobei in dem Zwischenbereich ein lichtundurchlässiges Element angeordnet ist.

2. Vorrichtung nach Anspruch 1, **dadurch gekennzeichnet, dass** das Lichtleitelement derart ausgebildet ist, dass die erste Oberfläche (3) mindestens einen Auskoppelbereich aufweist, wobei nur in dem mindestens einen Auskoppelbereich Licht aus dem Lichtleitelement auskoppelbar ist.

3. Vorrichtung nach Anspruch 2, **dadurch gekennzeichnet, dass** das Lichtleitelement eine weitere Oberfläche (4) aufweist, wobei die weitere Oberfläche (4) eine der ersten Oberfläche (3) gegenüberliegende Oberfläche des Lichtleitelements ist, wobei der mindestens eine Auskoppelbereich durch eine Ausbildung der weiteren Oberfläche (4) festgelegt ist.

4. Vorrichtung nach Anspruch 3, **dadurch gekennzeichnet, dass** eine
Oberflächenstruktur der weiteren Oberfläche (4) derart ausgebildet ist, dass nur in mindestens einem vorbestimmten Bereich der weiteren Oberfläche (4) das mittels der Beleuchtungseinrichtung (7) eingekoppelte Licht derart in Richtung der ersten Oberfläche (3) lenkbar ist, dass es durch die erste Oberfläche (3) austritt.

5. Vorrichtung nach einem der Ansprüche 1 bis 4, **dadurch gekennzeichnet, dass** das Lichtleitelement im Bereich einer weiteren Oberfläche (4) mindestens ein optisches Element aufweist oder ausbildet.

6. Vorrichtung nach einem der Ansprüche 1 bis 5, **dadurch gekennzeichnet, dass** die Auskoppelbereiche und/oder die erhabenen Bereiche (5) derart angeordnet sind, dass eine optische Erfassung mit einer vorbestimmten Auflösung möglich ist.

7. Vorrichtung nach einem der Ansprüche 1 bis 6, **dadurch gekennzeichnet, dass** das Lichtleitelement ein maximale Höhe aufweist.

8. Verfahren zur Erfassung einer Oberflächenstruktur eines Prüfkörpers, insbesondere eines Fingers (9), wobei der Prüfkörper auf eine erste Oberfläche (3) eines Lichtleitelements aufgelegt wird, wobei Licht in das Lichtleitelement eingekoppelt wird, wobei Licht im Bereich der ersten Oberfläche (3) aus dem Lichtleitelement hin zum Prüfkörper ausgekoppelt wird, wobei ein nicht an der ersten Oberfläche (3) reflektierter Anteil des Lichts optisch erfasst wird, **dadurch gekennzeichnet, dass** eine weitere Oberfläche (4)
des Lichtleitelements (3) mindestens zwei erhabene Bereiche (5) aufweist, wobei zwischen den mindestens zwei erhabenen Bereichen ein Zwischenbereich angeordnet ist,
das Lichtleitelement im Bereich der ersten Oberfläche (3) bei einem Luft-Lichtleitelement-Übergang für zumindest einen Teil des eingekoppelten Lichts vollständig reflektierend und bei einem Medium-Lichtleitelement-Übergang unvollständig reflektierend ist, wobei das Medium ein von Luft verschiedenes Medium ist, wobei in dem Zwischenbereich ein lichtundurchlässiges Element angeordnet ist.

9. Verfahren nach Anspruch 8, **dadurch gekennzeichnet, dass** Licht nur in mindestens einem Auskoppelbereich der ersten Oberfläche (3) aus dem Lichtleitelement ausgekoppelt wird.

10. Verfahren nach einem der Ansprüche 8 oder 9, **dadurch gekennzeichnet, dass** in Abhängigkeit des erfassten Anteils eine Lebenderkennung durchgeführt wird.

11. Verfahren zur Herstellung einer Vorrichtung (1) zur Erfassung einer Oberflächenstruktur eines Prüfkörpers, insbesondere eines Fingers (9), wobei mindestens eine Beleuchtungseinrichtung (7) und mindestens ein Lichtleitelement bereitgestellt wird, wobei die Beleuchtungseinrichtung (7) und das mindestens eine Lichtleitelement derart optisch verbunden werden, dass Licht in das Lichtleitelement einkoppelbar ist, wobei das Lichtleitelement derart bereitgestellt wird, dass das Lichtleitelement eine erste Oberfläche (3) aufweist, wobei Licht im Bereich der ersten Oberfläche (3) aus dem Lichtleitelement auskoppelbar ist, **dadurch gekennzeichnet, dass** eine weitere
Oberfläche (4) des Lichtleitelements (3) mindestens zwei erhabene Bereiche (5) aufweist, wobei zwischen den mindestens zwei erhabenen Bereichen ein Zwischenbereich angeordnet ist,
das Lichtleitelement im Bereich der ersten Oberfläche (3) für einen Luft-Lichtleitelement-Übergang für zumindest einen Teil des von der Beleuchtungseinrichtung (7) erzeugten Lichts vollständig reflektierend und bei einem Medium-Lichtleitelement-Übergang unvollständig reflektierend ist, wobei das Medium ein von Luft verschiedenes Medium ist, wobei in dem Zwischenbereich ein lichtundurchlässiges Element angeordnet ist.

12. Verfahren nach Anspruch 11, **dadurch gekennzeichnet, dass** die weitere Oberfläche (4) des Lichtleitelements bereitgestellt wird, wobei mindestens ein Auskoppelbereich der ersten Oberfläche (3) durch eine Ausbildung der weiteren Oberfläche (4) festgelegt ist.

## Claims

1. Device for optically detecting a surface structure of a test body, in particular of a finger (9), wherein the device (1) comprises at least one illumination device (7), wherein the device (1) comprises at least one light guiding element, wherein light can be coupled into the light guiding element by means of the illumination device (7), wherein the light guiding element has a first surface (3), wherein light can be coupled out of the light guiding element in the region of the first surface (3), **characterized in that** a further surface (4) of the light guiding element (3) has at least two raised regions (5), an intermediate region being arranged between the at least two raised regions, the light guiding element is completely reflective in the region of the first surface (3) for an air-light guiding element-transition for at least part of the light generated by the illumination device (7) and is incompletely reflective in the case of a medium-light guiding element-transition, the medium being a medium other than air, wherein an opaque element is arranged in the intermediate region.

2. Device according to claim 1, **characterized in that** the light guiding element is designed in such a way that the first surface (3) has at least one uncoupling region, wherein light can be coupled out of the light guiding element only in the at least one uncoupling region.

3. Device according to claim 2, **characterized in that** the light guiding element has a further surface (4), the further surface (4) being a surface of the light guiding element opposite the first surface (3), the at least one uncoupling region being defined by a formation of the further surface (4).

4. Device according to claim 3, **characterized in that** a surface structure of the further surface (4) is designed in such a way that the light coupled in by means of the illumination device (7) can be directed in the direction of the first surface (3) in such a way that it emerges through the first surface (3) only in at least one predetermined region of the further surface (4).

5. Device according to one of claims 1 to 4, **characterized in that** the light guiding element has or forms at least one optical element in the region of a further surface (4).

6. Device according to any one of claims 1 to 5, **characterized in that** the uncoupling regions and/or the raised regions (5) are arranged in such a way that optical detection with a predetermined resolution is possible.

7. Device according to one of claims 1 to 6, **characterized in that** the light guiding element has a maximum height.

8. Method for detecting a surface structure of a test body, in particular of a finger (9), wherein the test body is placed on a first surface (3) of a light guiding element, wherein light is coupled into the light guiding element, wherein light is coupled out of the light guiding element towards the test body in the region of the first surface (3), wherein a portion of the light not reflected at the first surface (3) is optically detected, **characterized in that** a further surface (4) of the light guiding element (3) has at least two raised regions (5), an intermediate region being arranged between the at least two raised regions, the light guiding element is completely reflective in the region of the first surface (3) for an air-light guiding element-transition for at least part of the light coupled in and is incompletely reflective in the case of a medium-light guiding element-transition, the medium being a medium other than air, wherein an opaque element is arranged in the intermediate region.

9. Method according to claim 8, **characterized in that** light is only coupled out of the light guiding element in at least one uncoupling region of the first surface (3).

10. Method according to one of claims 8 or 9, **characterized in that** a life detection is carried out as a function of the detected portion.

11. Method for producing a device (1) for detecting a surface structure of a test body, in particular a finger (9), wherein at least one illumination device (7) and at least one light guiding element are provided, wherein the illumination device (7) and the at least one light guiding element are optically connected in such a way that light can be coupled into the light guiding element, wherein the light guiding element is provided in such a way that the light guiding element has a first surface (3), wherein the light guiding element is provided in such a way that the light guiding element has a first surface (3), wherein light can be coupled out of the light guiding element in the region of the first surface (3), **characterized in that** a further surface (4) of the light-guiding element (3) has at least two raised regions (5), an intermediate region being arranged between the at least two raised regions, the light guiding element is completely reflective in the region of the first surface (3) for an air-light guiding element-transition for at least part of the light generated by the illumination device (7) and is incompletely reflective in the case of a medium-light guiding element-transition, the medium being a medium other than air, an opaque element being arranged in the intermediate region.

12. Method according to claim 11, **characterized in that** the further surface (4) of the light guiding element is provided, at least one uncoupling region of the first surface (3) being defined by a formation of the further surface (4).

## Revendications

1. Dispositif de détection optique d'une structure de surface d'un corps d'essai, en particulier d'un doigt (9), le dispositif (1) comprenant au moins un dispositif d'éclairage (7), le dispositif (1) comprenant au moins un élément de guidage de lumière, de la lumière pouvant être injectée dans l'élément de guidage de lumière au moyen du dispositif d'éclairage (7), l'élément de guidage de lumière présentant une première surface (3), la lumière pouvant être extraite de l'élément de guidage de lumière dans la zone de la première surface (3), **caractérisé en ce que** une autre surface (4) de l'élément de guidage de lumière (3) présente au moins deux zones en relief (5), une zone intermédiaire étant disposée entre les au moins deux zones en relief, l'élément de guidage de lumière est complètement réfléchissant dans la zone de la première surface (3) pour une transition air-élément de guidage de lumière pour au moins une partie de la lumière générée par le dispositif d'éclairage (7) et incomplètement réfléchissant pour une transition milieu-élément de guidage de lumière, le milieu étant un milieu différent de l'air, un élément opaque étant disposé dans la zone intermédiaire.

2. Dispositif selon la revendication 1, **caractérisé en ce que** l'élément de guidage de lumière est conçu de telle sorte que la première surface (3) présente au moins une zone de découplage, la lumière ne pouvant être découplée de l'élément de guidage de lumière que dans l'au moins une zone de découplage.

3. Dispositif selon la revendication 2, **caractérisé en ce que** l'élément de guidage de lumière présentant une autre surface (4), l'autre surface (4) étant une surface de l'élément de guidage de lumière opposée à la première surface (3), l'au moins une zone de découplage étant formée par une configuration de l'autre surface (4).

4. Dispositif selon la revendication 3, **caractérisé en ce qu'**une structure de surface de l'autre surface (4) est conçue de telle sorte que ce n'est que dans au moins une zone prédéterminée de l'autre surface (4) que la lumière couplée au moyen du dispositif d'éclairage (7) peut être dirigée en direction de la première surface (3) de telle sorte qu'elle sort par la première surface (3).

5. Dispositif selon l'une quelconque des revendications 1 à 4, **caractérisé en ce que** l'élément de guidage de lumière présente ou forme au moins un élément optique dans la zone d'une autre surface (4).

6. Dispositif selon l'une quelconque des revendications 1 à 5, **caractérisé en ce que** les zones de découplage et/ou les zones en relief (5) sont disposées de manière à permettre une détection optique avec une résolution prédéterminée.

7. Dispositif selon l'une des revendications 1 à 6, **caractérisé en ce que** l'élément de guidage de lumière présente une hauteur maximale.

8. Procédé de détection d'une structure de surface d'un corps d'essai, en particulier d'un doigt (9), le corps d'essai étant posé sur une première surface (3) d'un élément de guidage de lumière, de la lumière étant injectée dans l'élément de guidage de lumière, de la lumière étant découplée de l'élément de guidage de lumière vers le corps d'essai dans la zone de la première surface (3), une partie de la lumière non réfléchie sur la première surface (3) étant détectée optiquement, **caractérisé en ce qu'**une autre surface (4) présente au moins deux zones en relief (5), une zone intermédiaire étant disposée entre les au moins deux zones en relief, l'élément de guidage de lumière est complètement réfléchissant dans la zone de la première surface (3) pour au moins une partie de la lumière couplée dans le cas d'une transition air-élément de guidage de lumière et incomplètement réfléchissant dans le cas d'une transition milieu-élément de guidage de lumière, le milieu étant un milieu différent de l'air, un élément opaque étant disposé dans la zone intermédiaire.

9. Procédé selon la revendication 8, **caractérisé en ce que** la lumière n'est extraite de l'élément de guidage de lumière que dans une zone de découplage de la première surface (3).

10. Procédé selon l'une des revendications 8 ou 9, **caractérisé en ce qu'**une détection de vie est effectuée en fonction de la proportion détectée.

11. Procédé de fabrication d'un dispositif (1) de détection d'une structure de surface d'un corps d'essai, en particulier d'un doigt (9), au moins un dispositif d'éclairage (7) et au moins un élément de guidage de lumière étant mis à disposition, le dispositif d'éclairage (7) et le au moins un élément de guidage de lumière sont reliés optiquement de telle sorte que la lumière peut être couplée dans l'élément de guidage de lumière, l'élément de guidage de lumière étant mis à disposition de telle sorte que l'élément de guidage de lumière présente une première surface (3), la lumière pouvant être couplée hors de l'élément de guidage de lumière dans la zone de la première surface (3), caractérisé, qu'une autre surface (4) de l'élément de guidage de lumière (3) présente au moins deux zones en relief (5), une zone intermédiaire étant disposée entre les au moins deux zones en relief, l'élément de guidage de lumière est complètement réfléchissant dans la zone de la première surface (3) pour une transition air-élément de guidage de lumière pour au moins une partie de la lumière générée par le dispositif d'éclairage (7) et incomplètement réfléchissant pour une transition milieu-élément de guidage de lumière, le milieu étant un milieu différent de l'air, un élément opaque étant disposé dans la zone intermédiaire.

12. Procédé selon la revendication 11, **caractérisé en ce que** l'autre surface (4) de l'élément de guidage de lumière est fournie, au moins une zone de découplage de la première surface (3) étant définie par une formation de l'autre surface (4).
